Europäisches Patentamt

European Patent Office

Office européen des brevets

(19)

(11) Veröffentlichungsnummer : **0 105 211**
**B1**

(12) # EUROPÄISCHE PATENTSCHRIFT

(45) Veröffentlichungstag der Patentschrift :
28.05.86

(21) Anmeldenummer : 83108706.9

(22) Anmeldetag : 03.09.83

(51) Int. Cl.⁴ : **G 01 N 33/52, G 01 N 21/03, G 01 N 31/22**

(54) **Küvette zur Bestimmung chemischer Verbindungen in Flüssigkeiten.**

(30) Priorität : 11.09.82 DE 3233809

(43) Veröffentlichungstag der Anmeldung :
11.04.84 Patentblatt 84/15

(45) Bekanntmachung des Hinweises auf die Patenterteilung : 28.05.86 Patentblatt 86/22

(84) Benannte Vertragsstaaten :
AT BE CH DE FR GB IT LI LU NL SE

(56) Entgegenhaltungen :
EP-A- 0 054 679
DE-A- 2 015 271
DE-B- 2 215 089
FR-A- 2 325 920

(73) Patentinhaber : Boehringer Mannheim GmbH
Sandhofer Strasse 116
D-6800 Mannheim 31 (DE)

(72) Erfinder : Vogel, Peter, Dr. rer.nat.
Schubertweg 5
D-6944 Hemsbach (DE)
Erfinder : Rittersdorf, Walter, Dr. rer.nat.
Kasseler Strasse 6
D-6800 Mannheim 31 (DE)

EP 0 105 211 B1

Jouve, 18, rue St-Denis, 75001 Paris, France

**Beschreibung**

Die Erfindung betrifft eine Küvette sowie ihre Verwendung zur analytischen Bestimmung chemischer Verbindungen in Flüssigkeiten, durch spektralphotometrische Auswertung.

Die spektralphotometrische Analyse ist eine der in der Industrie am häufigsten angewandte Untersuchungsmethode von Flüssigkeiten. Reinheitsprüfungen, Qualitätsüberwachung, Reaktionssteuerung und Überwachung, qualitative und quantitative Analysen sind übliche Anwendungsgebiete. In der Klinischen Chemie ist es üblich, zur Erkennung von Krankheiten des menschlichen Körpers die Konzentration von bestimmten Verbindungen in Blut, Serum und Urin zu bestimmen.

Hierfür sind seit langem Verfahren bekannt, die unter Verwendung verschiedener Reagenzien und meist nach Verdünnen des Probenmaterials, die zu bestimmende Substanz in Transmissionsphotometern vermessen. Für diese meist nur stationär einzusetzenden Verfahren werden üblicherweise Glas- oder Kunststoffküvetten in der Standardschichtdicke 1 cm verwendet. In Ausnahmefällen werden auch Küvetten mit anderer Schichtdicke verwendet.

Aus dem für die Transmissionsphotometrie anzuwendenden Lambert-Beerschen Gesetz

$$E = \varepsilon \cdot c \cdot d$$

läßt sich leicht entnehmen, daß bei Messungen im Extinktionsbereich $E = 0,1\text{-}1$ und üblichen Substratkonzentrationen von $c = 10^{-5}\text{-}10^{-4}$ mol/l mit einem molaren Extinktionskoeffizienten $\varepsilon = 10^4$ mol$^{-1} \cdot$ l $\cdot$ cm$^{-1}$ eine Schichtdicke von ca. 1 cm entsprechend der Standardküvettendicke benötigt wird. Probleme treten dann auf, wenn die zu bestimmenden Substanzen in höheren Konzentration vorkommen. Üblicherweise wird in diesem Falle sie Probe verdünnt.

Schon seit längerem besteht in der analytischen Chemie die Tendenz, zur Vermeidung von Pipettier- und Verdünnungsfehlern unverdünntes Probenmaterial für analytische Zwecke einzusetzen. Weiterhin wird versucht, das benötigte Probenvolumen möglichst klein zu halten, da häufig nur geringe Mengen zur Verfügung stehen, aber auch, um aus Kostengründen die Reagenzienmenge zu begrenzen. Eine der Lösungen dieses Problems sind die seit Jahren bekannten Teststreifen, die die für die Reaktion notwendigen Chemikalien enthalten und durch Eintauchen beziehungsweise Tüpfeln mit kleinen Probenvolumina der zu untersuchenden Flüssigkeit analytische Verwendung finden, wobei die Konzentration der zu bestimmenden Substanz durch Vergleich mit einer Farbskala oder durch remissionsphotometrische Messung der Reaktionsfarben bestimmt wird. Häufig liefern Teststreifen aber aufgrund des inhomogenen Trägermaterials (Papiere) nicht die notwendige Präzision oder, wie zum Beispiel bei Verwendung von wasserfesten transparenten Filmen (z. B. DE-A-15 98 153) kann die notwendige Farbintensität nicht in allen Fällen erreicht werden. Ein zusätzliches Problem dieser Schnellteste auf Basis von saugfähigen Trägern oder Filmen besteht darin, daß die Substratdosierung sehr genau erfolgen muß oder aber, daß bei ungenauer Dosierung ein Überschuß entfernt werden muß. Eine weitere denkbare Lösung bei kleinen Volumina bietet die Verkleinerung der Schichtdicke der Küvette, was mit starren Materialien, wie zum Beispiel Glas oder Duroplasten (wie in der DE-A-27 29 294 beschrieben) zwar technisch möglich, aber aufwendig und nur mit hohen Kosten zu realisieren ist. Kostengünstig zu fertigende Wegwerfartikel sind dagegen schwierig mit der notwendigen Toleranz der Küverttendicke von 5 % zu fertigen.

Aus der US Re 29725 ist es bekannt, Reagenzien und Probe in einem Analysenbehälter der aus zwei am Rand miteinander verbundenen flexiblen Folien besteht, zu vermischen und zur Reaktion zu bringen und unter anderem der Reaktionsprodukt spektral photometrisch zu vermessen. Dazu wird der Analysenbehälter zwischen zwei planparalle, in definiertem Abstandzueinander im Strahlengang des Spektrometers angeordnete « Backen » gelegt und durch Druck auf den Äußeren Bereich des Analysenbehälters der Innendruck solange erhöht, bis sich die transparenten Wände glatt an die « Backen » anlegen. Der Abstand der « Backen » minus der zweifachen Dicke der Folie definiert dann die Schichtdicke der « Küvette ». Wiederum ist es schwierig, die Folien mit einer Toleranz von weniger als 5 % in der Dicke zu fertigen. Da dieser Fehler zweifach in die Schichtdicke der Küvette eingeht, können Schichtdicken unter 1 mm nur noch ungenau realisiert werden.

Das Ziel der der Erfindung zugrundeliegenden Aufgabe bestand darin, ein Verfahren zu entwickeln, welches die Bestimmung chemischer Verbindungen in Flüssigkeiten gestattet und sich einer Vorrichtung bedient, die die Vorzüge der Reagenzienpräsenz von Teststreifen mit der analytischen Präzision bei Verwendung von definierten Küvettendicken bei gleichzeitig geringen Probenvolumen vereint.

Es wurde nun gefunden, daß diese Aufgabe mit einem Verfahren gelöst werden kann, das für die spektralphotometrische Vermessung von Substanzen eine Küvette verwendet, deren Küvettenkammer durch ein Geflecht (Gewebe oder Gewirke) aus transparenten Fäden gebildet wird (im folgenden Abstandshalter genannt) wobei die Küvettenwände entweder aus zwei transparenten Formteilen oder einem transparenten Formteil und einem lichtreflektierenden Formteil bestehen. Die Dicke der Küvettenkammer wird durch die Dicke des Abstandshalters vorgebildet. Die Probe wird über eine seitliche Öffnung durch die Kapillaraktivität des Abstandshalters in die Küvettenkammer gebracht. Es wurde weiterhin gefunden, daß die für eine chemische Reaktion notwendigen Reagenzien entweder auf die Oberfläche einer oder beider Folien, die die Küvette begrenzen oder auf den Abstandshalter aufgebracht werden können oder aber in einer vor der Küvettenöffnung befindlichen Probenaufnahme- und Reaktionszone fixiert werden können, wobei das Probenmaterial vor Eintritt in die Küvette durch die Reaktionszone geführt wird.

Überraschenderweise wurde nun gefunden, daß bei Verwendung von monofilen Geweben für den Abstandshalter, wie sie zum Beispiel für die Verwendung als Siebmaterialien in großen Mengen hergestellt werden, mit Probenvolumen von wenigen µl bei Wiederholungsmessungen Variationskoeffizienten von 1 % erhalten werden können, obwohl die Fadenstärken der einzelnen Fasern um 5 % differieren solange die Anzahl der Fäden pro cm Gewebe konstant gehalten wird. Obwohl durch die Kapillaraktivität des Abstandshalters die Formteile der Küvette im gefüllten Zustand bereits relativ eng anliegen, ist es vorteilhaft, während der Messung einen leichten Außendruck auf die Formteile auszuüben und so ein enges Anliegen an dem Abstandshalter und damit eine konstante Schichtdicke der Küvette zu garantieren. Die Formteile werden vorzugsweise ebenfalls aus Kunststoffolien gefertigt.

Diese überraschende Konstanz der Messungen wird von uns so erklärt, daß für die Messung nicht der gesamte Raum zwischen den Fomteilen relevant ist, sondern nur der zwischen den Formteilen und den Fäden des Abstandshalters verbleibende Raum, der eine scheinbare Schichtdicke der Küvette definiert. Diese scheinbare Schichtdicke wird durch Materialtoleranzen weniger beeinflußt als es der effektiven Toleranz entspricht, da einerseits ein Bereich über mehrere Fäden zur Messung benutzt wird, so daß sich Fehler bereits kompensieren und andererseits zum Beispiel eine Vergrößerung des Formteilabstandes durch dickere Fäden gleichzeitig eine Verringerung der Hohlräume im Abstandshalter bewirkt, weil die Fäden enger zusammenliegen, so daß sich diese Änderungen weitgehend gegenseitig kompensieren.

Diese Kunststoffküvetten können sehr einfach in größerer Stückzahl gefertigt werden, indem die Materialien in bandförmiger Form an einer oder beiden Längskanten miteinander verklebt und anschließend in der geeigneten Breite quer dazu Streifen geschnitten werden.

Das den Abstandshalter bildende Geflecht besteht aus nicht saugfähigem in der zu untersuchenden Probe unlöslichem Material, das transparent sein und nur geringe Adsorption aufweisen soll. Das Geflecht kann aus einzelnen Fäden (monofil) oder aus gedrehten Fäden, die aus mehreren dünnen Einzelfäden bestehen (multifil), gewebt oder gewirkt sein. Vorzugsweise ist das Geflecht in Form eines monofilen Gewebes aufgebaut. Multifile Geflechte sind weniger geeignet, da sich die Fadenform unter Druck stärker ändern kann.

Das Material des Geflechts kann dabei aus wasserunlöslichen Polymeren oder Polykondensaten wie zum Beispiel Polyester, Polyamid, Polycarbonat, Polystyrol, Polyurethan und anderen bestehen. Glasfasern wären an sich wegen ihrer Inertheit besonders günstig, jedoch sind Gewebe mit geeigneten Dicken und Toleranzen nicht im Handel. Die Fäden, aus denen das Gewebe aufgebaut ist, können von ca. 10 µm-200 µm dick sein. Wenn es sich um ein monofiles Gewebe handelt, liegt die bevorzugte Dicke zwischen 20 µm und 100 µm. Die Fadendichte beträgt dabei zwischen 500 und 20 pro cm Gewebe.

Die Dicke des Geflechts hängt von der Fadendicke und von der Gewebeart ab und kann zwischen 30 µm oder 500 µm liegen, vorzugsweise aber zwischen 40 µm und 200 µm. Bei normalen Geweben entspricht sie der Dicke der Kreuzungspunkte, das heißt der Dicke von 2 Fäden.

Die transparenten Formteile sollen aus planem, nicht saugfähigem, in der Probe unlöslichem Material sein. Es kann aus Glas, aber auch aus einer Kunststoffolie, die zum Beispiel dem Material des Geflechts entspricht, bestehen. Die Dicke des Verwendeten Materials ist nicht kritisch, da sie in die Schichtdicke der Küvette, im Gegensatz zur Vorrichtung nach US Re 29725, nicht eingeht. Aus praktischen Gründen liegt sie zwischen 0,05 und etwa 1 mm.

Die lichtreflektierende Begrenzung der Küvette kann aus Metall, Glas, Keramik, Kunststoff und anderen reflektierenden Materialien bestehen, wobei die Lichtreflexion durch Verspiegeln, Oberflächenglanz oder durch Pigmentierung des Materials zustande kommen kann. Die der Küvette zugekehrte Seite soll plan, undurchlässig und nicht absorbierend sein. Die Dicke des Küvettenraums wird durch die Dicke des Geflechts vorgegeben. Wie oben gesagt, wird dies vorzugsweise erreicht, indem die Formteile durch die Optik des Spektralphotometers mit einem Druck von 0,1-10 bar zusammengedrückt werden, so daß während des Meßvorgangs die Küvettendicke einen konstanten, reproduzierbar durch die Materialbeschaffenheit des Geflechts verursachten Wert annimmt. Wenn die Formteile fest miteinander verbunden sind, muß mindestens eines der begrenzenden Materialien aus einem flexiblen Material bestehen oder aber die Material-Verbindungs-Stelle elastisch ausgebildet sein. Zweckmäßigerweise ist die Küvettean mindestens einer Seite offen, so daß ein vorhandener Probenüberschuß beim Zusammendrücken entweichen kann. Die Küvette kann durch Verkleben, Verschweißen oder Verklammern aller 3 Küvettenbauteile, mindestens aber der die Küvette begrenzenden Formteile in ihrer Form gehalten werden. Dies kann an einer Seite, an den gegenüberliegenden Seiten oder auf allen Seiten geschehen. Dabei kann das Befüllen mit dem zu vermessenden Probengut von einer oder mehreren Seiten, aber auch von hinten oder vorne durch entsprechende Öffnungen erfolgen.

Die Oberflächen von Abstandshalter und Formteilen sollten, um das Einfließen des Probengutes zu erleichtern, hydrophil sein oder entsprechend ausgerüstet sein. Weiterhin können die Oberflächen mit einem oder mehreren Reagenzien belegt sein, was durch Vorbehandlung wie Tauchen, Besprühen oder Bestreichen mit Netzmittel, beziehungsweise Reagenzienlösungen erfolgen kann.

In vielen Fällen ist es angebracht, vor der Messung beziehungsweise vor der in der Küvette

3

ablaufenden Reaktion aus der Probe gewisse Bestandteile abzutrennen oder unter anderen Bedingungen Vorreaktion ablaufen zu lassen. Dazu kann außerhalb des eigentlichen, für die Messung vorgesehenen Küvettenraums eine Probenaufnahmezone als separate Trenn- und/oder Reaktionszone vorgesehen sein, auf die die Probe aufgebracht wird und von der sie nach Ablauf der Trenn- beziehungsweise Vorreaktionsschritte in die Küvette, überführt wird. Am einfachsten kann diese Überführung durch einen kapillaraktiven Kontakt mit dem Abstandshalter bewirkt werden. In Spezialfällen, zum Beispiel wenn eine zeitlich definierte Vorreaktion ablaufen soll, kann die Übertragung auch aktiv, beispielsweise durch Zusammendrücken der Reaktionszone, erfolgen.

Zum Befestigen der Probeaufnahmezone wird mindestens eines der Formteile um ein entsprechendes Stück über die Länge des durch den Abstandshalter definierten Küvettenteils hinaus verlängert. Eine zusätzliche Verlängerung kann vorteilhaft als Handgriff dienen und Platz für eine Markierung oder Identifizierung bieten.

Die Trenn- oder Reaktionszone sollte aus saugfähigen Materialien wie zum Beispiel Papieren, Vliesen aus Kunststoff oder Kunststoff-Zelstoffgemischen oder anderen ähnlichen Materialien bestehen, die durch Besprühen oder Imprägnieren mit den notwendigen Reagenzien ausgerüstet sind. Soweit diese Reagenzien die nachfolgende Messung stören, können sie trägerfixiert sein.

Ionenaustauscherpapiere können zur Abtrennung von störenden Ionen, geeignete Filtermaterialien zur Abtrennung von korpuskulärem Material dienen. Ein Überschuß an Probe, der nicht von der Küvette aufgenommen wird, kann gleichfalls von dieser Zone aufgenommen werden und erlaubt ein undosiertes Auftragen der Probe.

Das erfindungsgemäße Verfahren kann im Einzelfall so variiert werden, daß die Vorrichtung aus einer Küvette besteht, bei der alle Materialien durchsichtig sind, so daß die Bestimmung im Durchlicht erfolgen kann, wobei die Lichtquelle auf der einen und der lichtempfindliche Detektor auf der anderen Seite der Küvette plaziert .sind. Die Bestimmung kann aber auch durch Reflexionsphotometrie erfolgen, wobei Lichtquelle und Photodetektor auf der gleichen Seite der Küvette angeordnet sind. Auch kann das erfindungsgemäße Verfahren ganz ohne chemische Reagenzien auskommen, wenn die zu analysierende Probe aus einer farbigen Lösung, wie zum Beispiel einem Färbebad oder einer Tinte besteht, oder wenn zum Beispiel die Konzentration der Erythrozyten des Bluts oder der Gehalt des Serums an Bilirubin gemessen werden soll.

Da im Gegensatz zu den vorbekannten Küvetten, deren Schichtdicke durch außerhalb des Strahlengangs liegende Maßnahmen bestimmt wird, (Seitenwände der Standardküvette, « Backen » gemäß US Re 29725) der Abstandshalter im Strahlengang liegt, sird außer im Spezialfall, in dem Faden und Probe gleichen Brechungsindex besitzen, zusätzliche Brechungen an den Fadenoberflächen zu berücksichtigen. Um Streuverluste zu vermeiden, muß deshalg die Empfängerseite des Spektrometers so ausgebildet sein, daß auch das Streulicht aufgefangen wird. Dazu muß direkt hinter der Küvette eine Sammellinse oder Ulbrichtkugel vorgesehen sein. Ansonsten können übliche Spektrometeranordnungen verwendet werden.

Weitere erfindungswesentliche Merkmale und Vorteile ergeben sich aus den nachfolgenden an Hand der Zeichnungen näher erläuterten Ausführungsbeispielen. Es zeigen :

Abb. 1 : Küvette mit Probenaufnahmezone (Querschnitt)

Abb. 2 : Küvette ohne besondere Probenaufnahmezone (Querschnitt)

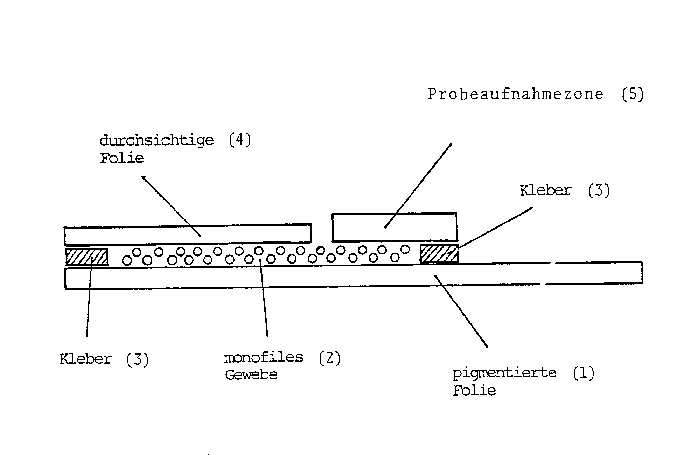

Abb. 1 zeigt eine pigmentierte Folie (1) die von einem monofilen Gewebe (2) überlagert ist. Mit einem Kleber (3) ist oberhalb des Gewebes (2) eine durchsichtige Folie (4) und eine Probenaufnahmezone (5) (z. B. ein Glasfaserflies) an der pigmentierten Folie (1) befestigt. Das Gewebe (2) wird ebenfalls durch mindestens eine dieser Klebestellen befestigt oder wird durch die Klemmwirkung der Folien (1), (4) und (5) gehalten. Die Probenaufnahmezone (5) ist in kapillarem Kontakt mit dem darunter befindlichen Gewebe (2). Überschüssige Probenflüssigkeit kann bei Druck auf die Folien (1) und (4) über die offenen Seiten austreten.

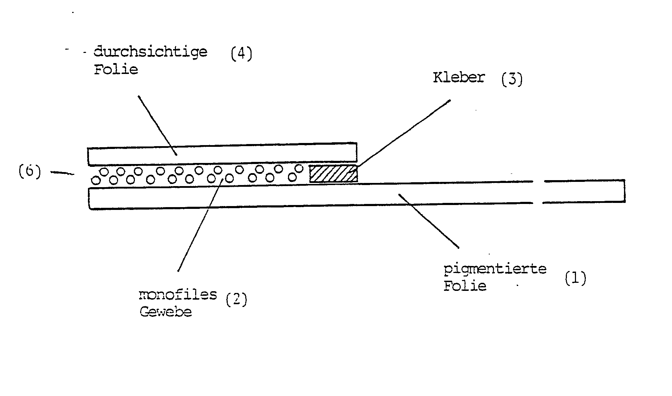

Abb. 2 zeigt eine pigmentierte Folie (1), die mit einem monofilen Gewebe (2) überlagert ist. Mit einem Kleber (3) ist oberhalb des Gewebes (2) eine durchsichtige Folie (4) befestigt. Die untere Kante (6) der Vorrichtung dient zur Aufnahme der flüssigen Probe.

Verwendungszweck, Herstellungsweise und die Handhabung sollen im einzelnen an einigen im folgenden beschriebenen Beispielen demonstriert werden.

Beispiel 1

Mittel zur Bestimmung von Hämoglobin in Blut

| | |
|---|---|
| 0,3 g | Dioctyl-Na-sulfosuccinat |
| 0,3 g | Diamyl-Na-sulfosuccinat |
| 2,5 g | Saponin |
| 2,0 g | rotes Blutlaugensalz |
| 9,0 g | Quecksilber (II)-cyanid |

werden in 100 ml 0,1 M Citratpuffer gelöst. Mit dieser Lösung wird ein 500 µm dickes Polyamid-Vlies (SL 4207 KA der Fa. Kalff) getränkt und anschließend bei 60 °C getrocknet. Das imprägnierte Vlies wird in Streifen von 6 mm Breite geschnitten.

Aus Streifen

einer mit $TiO_2$ pigmentierten Polystyrol-Folie, 500 μm dick, 77 mm breit

einer durchsichtigen Polycatbonatfolie, (Pokalon N, transparent der Fa. Lonza), 150 μm dick, 15 mm breit

einem monofilen Polyamidgewebe, (NY 15 HC, der Fa. Züricher Beuteltuchfabrik), Fadendicke 30 μ, Fadenzahl 222, das mit 0,05 % Dioctyl-Na-sulfosuccinat in Wasser gewaschen wurde, 15 mm breit

und dem imprägnierten Vlies

wird mit einem Schmelzkleber, der aus einer geheizten Düse aufgebracht wird, eine Anordnung gemäß Abbildung 1 zusammengeklebt. Die bandförmige Anordnung wird anschließend in 6 mm breite Streifen geschnitten. Auf die 6 × 6 mm große Probeaufnahmezone (Vlies) einer solchen Küvette werden ca. 25 μl Blut aufgetropft.

Das Blut fließt nun über das Reaktionsvlies, in dem Erythrozyten hämolysiert werden und das dadurch freigewordene Hämoglobin zu Hämoglobin-Cyanid umgesetzt wird, in den Küvettenspalt. Die Reaktionsfarbe des Hämoglobin-Cyanids wird am Remissionsphotometer PMQ III mit Kugelansatz KA von Zeiss bei 540 nm vermessen, wobei der Teststreifen mit der Seite der durchsichtigen Folie vor die Meßöffnung gebracht wird und mit einem plangeschliffenen Magneten angedrückt (0,5 bar) wird. Über eine zuvor mit Hämoglobin-Lösungen ermitelten Eichkurve können die Remissionswerte in Hämoglobin-Konzentration umgerechnet werden. Mit dieser Anordnung können im Konzentrationsbereich 25-200 g HB/l Hämoglobin-Bestoimmungen aus Blut mit einer Präzision von Tag zu Tag von 2 % (Variationskoeffizient) durchgeführt werden.

Tabelle I

| HB-Konz. g/l | Remission % |
|---|---|
| 19 | 55 |
| 57 | 35.1 |
| 85 | 28.5 |
| 123 | 23.4 |
| 140 | 21.2 |
| 174 | 17.9 |
| 209 | 15.8 |
| 233 | 14.3 |

Beispiel 2

Mittel zur Bestimmung von Bilirubin in Serum

Aus Streifen

einer pigmentierten Polystyrol-Folie von 500 μm Dicke, 77 mm breit

einer durchsichtigen Polyacetatfolie (Ultraphan®, Fa. Lonza), 200 μm dick, 15 mm breit

einem monofilen Polyestergewebe (PE 73 HC der Fa. Züricher Beuteltuchfabrik), Fadendicke 38 μm, Fadenzahl 89.5) 15 mm breit

und einem Glasfaservlies, 60 g/m², (Nr. 9 der Fa. Schleicher & Schüll), das mit einer Lösung von 5 % rotem Blutlaugensalz in Wasser imprägniert wurde, 6 mm breit wird eine Vorrichtung gemäß Beispiel 1 (Abbildung 1) zusammengeklebt und zu 6 mm breiten Streifen verarbeitet. 20 μl bilirubinhaltiges Vollblut werden auf das Probeaufnahme-Vlies aufgetropft. Das Glasfaservlies hält die Blutkörperchen zurück. Das Serum mit dem zu Biliverdin oxidiertem Bilirubin fließt in den Küvettenspalt und wird wie in Beispiel 1 am Remissionsphotometer PMQ 3 bei 630 nm vermessen. Die Bilirubin-Konzentration in Serum kann damit über eine Eichkurve bestimmt werden.

Beispiel 3

Mittel zur Bestimung der Konzentration von Evan's Blau in wäßrigen Lösungen

Aus Streifen

einer pigmentierten Polystyrolfolie von 500 μm Dicke, 77 mm breit

einer durchsichtigen Polyesterfolie, 100 μm, 15 mm breit

einem monofilen Polyamidgewebe (NY 64 HC der Fa. Züricher Beuteltuchfabrik), 15 mm breit.

wird eine Vorrichtung gemäß Abbildung 2 zusammengeklebt und zu Streifen von 10 mm Breite verarbeiten. Die Streifen werden mit der unteren Kante vorsichtig mit der Oberfläche einer wäßrigen Lösung von Evan's Blau in Berührung gebracht, wobei sich der Küvettenraum sofort mit der Lösung füllt. Die gefüllten Streifen werden am Remissionsphotometer PMQ 3 bei 640 nm mit Kugelansatz KA von Zeiss vermessen, die Remissionswerte können dann über eine Eichkurve in Konzentration umgerechnet werden. Bei Wiederholungsmessungen (n = 10) wird in Konzentrationsbereich 0.01-1.0 mmol/l ein Variationskoeffizient von ca. 1 % gemessen, bei einem Bias von 2 %.

Tabelle II

| Konzentration mmol/l | Remission % |
|---|---|
| 0.00 | 78 |
| 0.100 | 66 |
| 0.250 | 54 |
| 0.500 | 43 |
| 0.750 | 33 |
| 1.000 | 26,5 |

**Patentansprüche**

1. Küvette zur Bestimmung chemischer Verbindungen in Flüssigkeiten durch spektralphotometrische Auswertung, wobei die Küvette aus zwei zueinander parallelen, planen Formteilen besteht, wobei entweder beide Teile im zur Auswertung verwendeten Spektralbereich transparent oder ein Teil transparent und ein Teil reflektierend ist, dadurch gekennzeichnet, daß sich zwischen diesen Formteilen ein planer Abstandshalter aus einem Fadengeflecht, aus einem nicht saugfähigen, in der zu untersuchenden Probe unlöslichen, transparenten Material, befindet und die Länge des Lichtweges durch die Dicke des Abstandshalters und die Anzahl der Fäden pro cm Geflecht vorgegeben ist und die Formteile und der Abstandshalter an mindestens einer Kante miteinander verbunden sind.

2. Küvette nach Anspruch 1, dadurch gekennzeichnet, daß der Abstandshalter ein monofiles oder multifiles Gewebe aus Kunststoff-Fäden ist.

3. Küvette nach Anspruch 1 oder 2, dadurch gekennzeichnet, daß sich die für die Nachweisreaktion notwendigen Reagenzien in fester Form auf der dem Abstandshalter zugewandten Seite der Formteile und/oder auf dem Abstandshalter befinden.

4. Küvette nach Anspruch 1 oder 2, dadurch gekennzeichnet, daß neben dem Abstandshalter ein saugfähiges Material als Probenaufnahmezone angebracht ist.

5. Küvette nach Anspruch 4, dadurch gekennzeichnet, daß das saugfähige Material als Reaktions- und/oder Trennschicht ausgebildet ist.

6. Küvette nach Anspruch 4 oder 5, dadurch gekennzeichnet, daß das saugfähige Material von einem der Formteile nicht oder nur teilweise bedeckt ist.

7. Küvette nach einem der Ansprüche 1-6, dadurch gekennzeichnet, daß einer der Formteile über den Küvettenbereich hinaus verlängert und als Handgriff ausgebildet ist.

8. Küvette nach einem der Ansprüche 4-7, dadurch gekennzeichnet, daß das saugfähige Material der Probeaufnahmezone zusätzliche Reagenzien enthält.

9. Verwendung einer Küvette nach Anspruch 1-4 zur Bestimmung chemischer Verbindungen beziehungsweise biologischen Materialien in Körperflüssigkeiten wie Urin, Serum und Blut.

10. Verwendung einer Küvette nach Anspruch 1-4 zur Bestimmung von chemischen Verbindungen in wäßringen Flüssigkeiten.

11. Verwendung einer Küvette nach Anspruch 1-4 zur Bestimmung von Hämoglobin in Blut oder Bilirubin in Serum.

**Claims**

1. Cuvette for the determination of chemical components in liquids by spectrophotometric evaluation, whereby the cuvette consists of two planar shaped parts parallel to one another, whereby either both parts are transparent in the spectral range used for the evaluation or one part is transparent and one part reflecting, characterised in that between these shaped parts, there is present a planar distance piece of a filamentary reticulation of a non-absorbent transparent material insoluble in the sample to be investigated and the length of the light path being predetermined by the thickness of the distance piece and the number of filaments per cm of reticulation and the shaped parts and the distance piece are joined together on at least one edge.

2. Cuvette according to claim 1, characterised in that the distance piece is a monofilar or multifilar fabric of synthetic resin filaments.

3. Cuvette according to claim 1 or 2, characterised in that the reagents necessary for the detection reaction are present in solid form on the side of the shaped parts facing the distance piece and/or on the distance piece.

4. Cuvette according to claim 1 or 2, characterised in that, in addition to the distance piece, an absorbent material is provided as sample reception zone.

5. Cuvette according to claim 4, characterised in that the absorbent material is constructed as reaction and/or separation layer.

6. Cuvette according to claim 4 or 5, characterised in that the absorbent material is not covered or only partly covered by one of the shaped parts.

7. Cuvette according to one of claims 1-6, characterised in that one of the shaped parts is extended beyond the cuvette range and is constructed as a handle.

8. Cuvette according to one of claims 4-7, characterised in that the absorbent material of the sample reception zone contains additional reagents.

9. Use of a cuvette according to claims 1-4 for the determination for chemical compounds or biological materials in body fluids, such as urine, serum or blood.

10. Use of a cuvette according to claims 1-4 for the determination of chemical compounds in aqueous liquids.

11. Use of a cuvette according to claims 1-4 for the determination of haemoglobin in blood or of bilirubin in serum.

## Revendications

1. Cuvette pour le dosage de composés chimiques dans des liquides au moyen d'une évaluation spectrophotométrique, la cuvette étant composée de deux pièces préformées planes parallèles entre elles, ces deux pièces étant soit toutes deux transparentes dans le domaine spectral utilisé pour l'évaluation, soit une pièce est transparente et l'autre est réfléchissante, caractérisée en ce qu'il se trouve entre ces pièces préformées un écarteur plan constitué par un treillis de fils en une matière non absorbante transparente et insoluble dans l'échantillon à examiner, en ce que la distance parcourue par la lumière est prédéterminée par l'épaisseur de l'écarteur et par le nombre de fils par cm de treillis et en ce que les pièces préformées et l'écarteur sont reliés ensemble sur au moins un côté.

2. Cuvette selon la revendication 1, caractérisé en ce que l'écarteur est un tissu monofilaire ou multifilaire de fils synthétiques.

3. Cuvette selon la revendication 1ou 2, caractérisée en ce que les réactifs nécessaires pour la réaction de mise en évidence se trouvent sous forme solide sur le côté tourné vers l'écarteur de chacune des pièces préformées et/ou sur l'écarteur.

4. Cuvette selon la revendication 1 ou 2, caractérisée en ce qu'à côté de l'écarteur est placée une matière absorbante en tant que zone de réception d'échantillons.

5. Cuvette selon la revendication 4, caractérisée en ce que la matière absorbante a reçu la forme d'une couche de réaction et/ou de séparation.

6. Cuvette selon la revendication 4 ou 5, caractérisée en ce que la matière absorbante n'est pas recouverte ou seulement partiellement, par l'une des pièces préformées.

7. Cuvette selon l'une quelconque des revendications 1 à 6, caractérisée en ce que l'une des pièces préformées est prolongée au-delà du contour de la cuvette et revêt la forme d'une poignée.

8. Cuvette selon l'une quelconque des revendications 4 à 7, caractérisée en ce que la matière absorbante de la zone de réception des échantillons comporte des réactifs supplémentaires.

9. Utilisation d'une cuvette selon l'une quelconque des revendications 1 à 4 pour le dosage de composés chimiques ou de matières biologiques dans des liquides somatiques comme l'urine, le sérum et le sang.

10. Utilisation d'une cuvette selon l'une quelconque des revendications 1 à 4 pour le dosage de composés chimiques dans des liquides aqueux.

11. Utilisation d'une cuvette selon l'une quelconque des revendications 1 à 4 pour le dosage d'hémoglobine dans le sang ou de la bilirubine dans le sérum.

Probeaufnahmezone (5)

durchsichtige (4)
Folie

Kleber (3)

Kleber (3)

monofiles (2)
Gewebe

pigmentierte (1)
Folie

1

durchsichtige (4)
Folie

Kleber (3)

(6)

monofiles (2)
Gewebe

pigmentierte (1)
Folie